# EUROPEAN PATENT APPLICATION

(11) **EP 3 318 209 A1**
(43) Date of publication of application: **09.05.2018**
(21) Application number: 16832567.8
(22) Date of filing: 06.05.2016
(51) Int. Cl.: A61B 18/12, A61B 17/00

(54) **TREATMENT TOOL**

(30) Priority: 05.08.2015 JP 2015154770
(71) Applicant: Olympus Corporation, Hachioji-shi, Tokyo 192-8507 (JP)
(72) Inventor: KAGA, Tomoyuki, Hachioji-shi Tokyo 192-8507 (JP); MASUDA, Shinya, Hachioji-shi Tokyo 192-8507 (JP)
(74) Representative: Schicker, Silvia
(86) International application number: PCT/JP2016/063670
(87) International publication number: WO 2017/022287

(57) **Abstract**

A treatment tool (100) includes a sheath (162), a first grasping member (120), a second grasping member (130) and a swing member (140). The first grasping member (120) protrudes from a distal portion of the sheath (162). The second grasping member (130) is rotatably provided to the sheath (120) so that a first angle, which is an angle formed with the first grasping member, changes. The swing member (140) is provided to the second grasping member (130) so as to swing and so that a second angle, which is an angle formed with the second grasping member, changes. When the second angle is at its maximum, there is a first angle at which the first grasping member (120) and the swing member (140) do not contact each other.

## Description

### Technical Field

The present invention relates to a treatment tool for treating biological tissue by pinching the biological tissue with two members.

### Background Art

In general, there is known a treatment tool that pinches and holds biological tissue as a treatment object with two members to treat the biological tissue. For example, Jpn. Pat. Appln. KOKAI Publication No. 2009-160404 discloses a technique related to such a treatment tool. The treatment tool grasps biological tissue using a jaw and a probe that vibrates at an ultrasonic frequency. The treatment tool treats the biological tissue by the probe vibrating at the ultrasonic frequency, and by the probe and the jaw as a bipolar electrode supplying high-frequency power to the biological tissue. Also, Jpn. Pat. Appln. KOKAI Publication No. 2009-160404 discloses a mechanism in which a part of the jaw that contacts the biological tissue swings, and an angle of a face of the jaw that is opposite to the probe changes.

### Summary of Invention

In a treatment tool that grasps biological tissue with a pair of grasping members to treat the biological tissue and has a mechanism in which a part of the grasping member swings, as disclosed in Jpn. Pat. Appln. KOKAI Publication No. 2009-160404, it is not desirable for the part of the grasping member that swings to come into contact with the other grasping member unnecessarily due to the swing.

An object of the present invention is to provide a treatment tool that includes two grasping members with an optimized swing mechanism of the grasping members.

According to an aspect of the present invention, a treatment tool includes: a sheath that is provided in a manner that a longitudinal axis thereof is arranged on a virtual plane; a first grasping member that protrudes from a distal portion of the sheath along the longitudinal axis; a second grasping member that is rotatably provided to the sheath on the virtual plane so that a first angle, which is an angle formed with the first grasping member, changes within a predetermined first range; a swing member that is provided to the second grasping member so as to swing on the virtual plane and so that a second angle, which is an angle formed with the second grasping member, changes within a predetermined second range; and an actuating member that is disposed along the sheath, is connected to the second grasping member, and moves along the longitudinal axis to thereby displace the second grasping member so as to change the first angle, wherein when the second angle is at its maximum within the second range, there is a first angle at which the first grasping member and the swing member do not contact each other within the first range.

According to the present invention, it is possible to provide a treatment tool that includes two grasping members with an optimized swing mechanism of the grasping members.

### Brief Description of Drawings

FIG. 1 is a schematic view of a configuration example of a surgical system according to one embodiment.
FIG. 2 is a schematic side view of a configuration example of a treatment portion according to the embodiment.
FIG. 3 is a schematic perspective view of a configuration example of a treatment portion according to the embodiment.
FIG. 4 is a schematic diagram of a configuration example of a treatment portion according to the embodiment.
FIG. 5 is a schematic diagram of a configuration example of a treatment portion according to the embodiment.
FIG. 6 is a schematic diagram of a configuration example of a treatment portion according to the embodiment.
FIG. 7 is a schematic diagram of a configuration example of a treatment portion according to a comparative example.
FIG. 8 is a schematic perspective view of a configuration example of a treatment portion according to a first modification.
FIG. 9 is a schematic diagram of a configuration example of a treatment portion according to a second modification.
FIG. 10 is a schematic perspective view of a configuration example of a treatment portion according to a third modification.
FIG. 11 is a schematic diagram of a configuration example of a treatment portion according to the embodiment.
FIG. 12 is a schematic diagram of a configuration example of a treatment portion according to the third modification.
FIG. 13 is a schematic perspective view of a configuration example of a treatment portion according to a fourth modification.
FIG. 14 is a schematic diagram of a configuration example of a treatment portion according to the fourth modification.

### Description of Embodiments

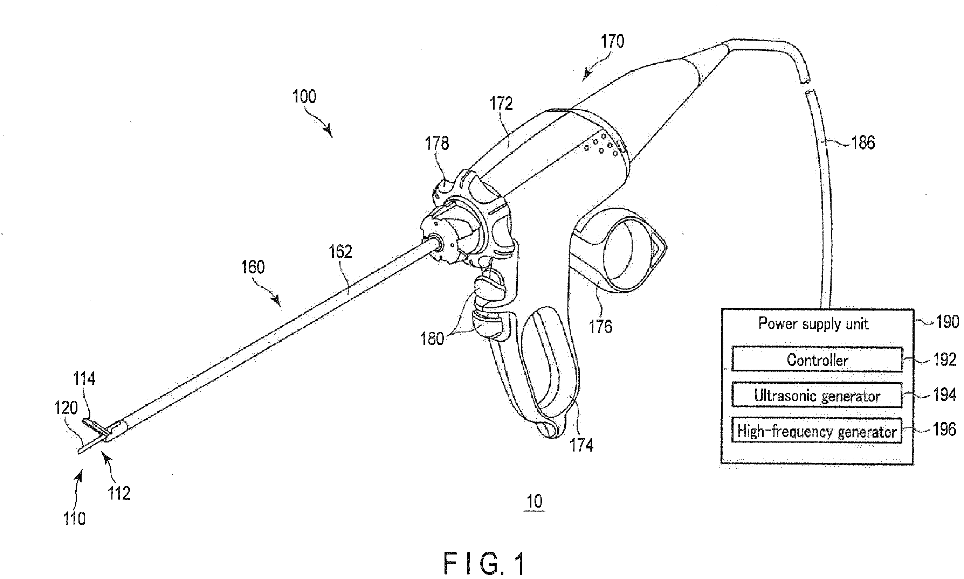

An embodiment of the present invention will be described with reference to the drawings. FIG. 1 schematically illustrates a surgical system 10 according to the present embodiment. The surgical system 10 includes a treatment tool 100 and a power supply unit 190, as shown in the figure.

The treatment tool 100 includes a treatment portion 110, a shaft 160, and an operating portion 170. In the description provided below, a side of the treatment portion 110 is referred to as a distal side, and a side of the operating portion 170 is referred to as a proximal side. The surgical system 10 is configured to grasp, with the treatment portion 110, biological tissue, such as membranous tissue, organ, bone, and blood vessel, which is to be treated. The surgical system 10 cuts the biological tissue grasped by the treatment portion 110 by using ultrasonic vibration while, for example, sealing the biological tissue. The surgical system 10 applies a high-frequency voltage to the grasped biological tissue, to seal or coagulate the biological tissue.

The shaft 160 includes a hollow sheath 162. A probe 112 that transmits ultrasonic waves and vibrates in a longitudinal direction is disposed in the sheath 162. A proximal end of the probe 112 is located in the operating portion 170. A distal side of the probe 112 protrudes from the sheath 162 and is located in the treatment portion 110. The distal side of the probe 112 constitutes a first grasping member 120.

A jaw 114 is provided in the treatment portion 110. The jaw 114 opens and closes relative to the first grasping member 120 being a distal portion of the probe 112. By this opening and closing, the first grasping member 120 and the jaw 114 grasp biological tissue as a treatment object. A part of the first grasping member 120 and a part of the jaw 114 function as a bipolar electrode that applies a high-frequency voltage to the grasped biological tissue. A part of the first grasping member 120 or a part of the jaw 114 may function as a monopolar electrode.

The operating portion 170 is provided with an operating portion main body 172, a fixed handle 174, a movable handle 176, a rotating knob 178, and an output switch 180. An ultrasonic transducer unit is provided to the operating portion main body 172. The proximal side of the probe 112 is connected to the ultrasonic transducer unit. An ultrasonic transducer is provided to the ultrasonic transducer unit, and ultrasonic vibration generated by the ultrasonic transducer is transmitted by the probe 112. As a result, the first grasping member 120 vibrates in the longitudinal direction thereof, and the biological tissue grasped by the treatment portion 110 is cut. In this manner, the first grasping member 120 is configured so that the ultrasonic transducer is directly or indirectly connected thereto.

The fixed handle 174 is fixed to the operating portion main body 172, and the movable handle 176 changes its position relative to the operating portion main body 172. The movable handle 176 is connected to a wire or a rod that is inserted into the shaft 160. The wire or the rod is connected to an actuating member described below that is connected to the jaw 114. The operation of the movable handle 176 is transmitted to the jaw 114 via the wire or rod and the actuating member. The jaw 114 changes its position relative to the first grasping member 120 according to the operation of the movable handle 176. The rotating knob 178 is a knob for rotating a portion closer to the distal side than the rotating knob 178. The treatment portion 110 and the shaft 160 rotate according to the rotation of the rotating knob 178, so that an angle of the treatment portion 110 is adjusted.

The output switch 180 includes, for example, two buttons. One of the buttons is a button that is pressed when exerting high-frequency power and ultrasonic vibration on the biological tissue as a treatment object by the treatment portion 110. The power supply unit 190 detecting that this button is pressed applies a high-frequency voltage between the first grasping member 120 and the jaw 114, and drives the ultrasonic transducer. As a result, the biological tissue grasped by the treatment portion 110 is coagulated or sealed, and cut. The other button is a button that is pressed when exerting only high-frequency power on the biological tissue as a treatment object by the treatment portion 110. The power supply unit 190 detecting that this button is pressed applies a high-frequency voltage between the first grasping member 120 and the jaw 114, and does not drive the ultrasonic transducer. As a result, the biological tissue grasped by the treatment portion 110 is coagulated or sealed without being cut.

One end of a cable 186 is connected to the proximal side of the operating portion 170. The other end of the cable 186 is connected to the power supply unit 190. The power supply unit 190 includes a controller 192, an ultrasonic generator 194, and a high-frequency generator 196.

The controller 192 controls the operation of each component of the surgical system 10. The controller 192 includes one or more of, for example, a central processing unit (CPU), an application specific integrated circuit (ASIC), a field programmable gate array (FPGA), etc. The controller 192 operates, for example, according to a program stored in a storage unit or a storage region in the controller. For example, the controller 192 controls the operation of the ultrasonic generator 194 or the high-frequency generator 196 according to input from the output switch 180. The ultrasonic generator 194 drives the ultrasonic transducer under the control of the controller 192. The high-frequency generator 196 supplies a high-frequency current to the treatment portion 110 under the control of the controller 192.

Next, the operation of the surgical system 10 according to the present embodiment is described. An operator operates an input section of the power supply unit 190, to set, in advance, the output conditions of the treatment tool, such as output power of high-frequency energy and output power of ultrasonic energy. The surgical system 10 may be configured so that values of the conditions are individually set or a set of setting values according to an operative procedure is selected.

The treatment portion 110 and the shaft 160 are inserted, for example, through an abdominal wall into an abdominal cavity. The operator operates the movable handle 176 to open and close the treatment portion 110, and grasps biological tissue as a treatment object with the first grasping member 120 and the jaw 114. The operator operates the output switch 180 upon grasping the biological tissue with the treatment portion 110. For example, the controller 192 of the power supply unit 190, detecting that one of the two buttons of the output switch 180 is pressed, outputs a drive-related instruction to the ultrasonic generator 194 and the high-frequency generator 196.

The high-frequency generator 196 applies a high-frequency voltage to the first grasping member 120 and the jaw 114 of the treatment portion 110 under the control of the controller 192, to cause a high-frequency current to flow to the biological tissue as a treatment object. Since the biological tissue serves as an electric resistor when the high-frequency current flows therethrough, heat is generated in the biological tissue and the temperature of the biological tissue rises. At this time, the temperature of the biological tissue becomes, for example, approximately 100°C. As a result, protein is denatured, and the biological tissue is coagulated and sealed.

The ultrasonic generator 194 drives the ultrasonic transducer under the control of the controller 192. As a result, the first grasping member 120 vibrates at an ultrasonic frequency in the longitudinal direction thereof. Due to the heat of the friction between the biological tissue and the first grasping member 120, the temperature of the biological tissue rises. As a result, protein is denatured, and the biological tissue is coagulated and sealed. The effect of sealing the biological tissue by the ultrasonic vibration is smaller than the effect of sealing the biological tissue by the application of a high-frequency voltage. Also, the temperature of the biological tissue becomes, for example, approximately 200°C. As a result, the biological tissue breaks and is cut. In this manner, the biological tissue grasped by the treatment portion 110 is cut while being coagulated and sealed. The treatment of the biological tissue is then completed.

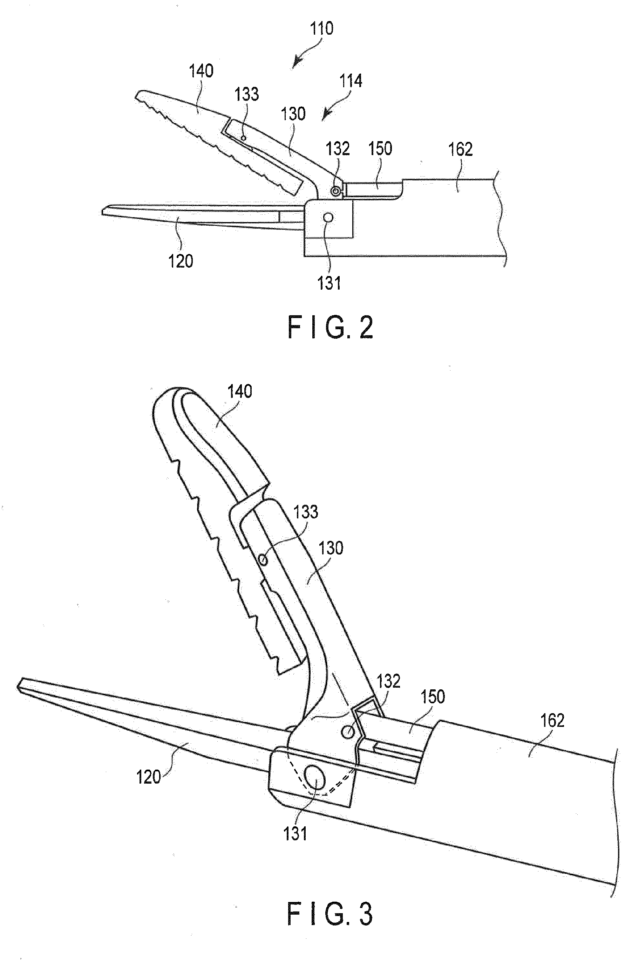

The configuration of the treatment portion 110 is described in detail below. FIG. 2 shows a lateral view of the treatment portion 110. FIG. 3 shows a perspective view of the treatment portion 110. The first grasping member 120, which is a distal portion of the probe 112, protrudes from a distal end of the sheath 162 constituting the shaft 160. The jaw 114 includes a second grasping member 130 and a swing member 140.

A first pivot 131 is provided at the distal portion of the sheath 162. A proximal portion of the second grasping member 130 is rotatably connected to the sheath 162 by the first pivot 131. In this manner, the sheath 162 and the second grasping member 130 are connected to each other so that an angle formed by the first grasping member 120 and the second grasping member 130 changes.

A second pivot 132 is provided at a portion of the proximal end of the second grasping member 130 that is slightly closer to the distal side than the first pivot 131. An actuating member 150 is connected to the second pivot 132 of the second grasping member 130. The actuating member 150 is a rod-shaped member disposed inside the sheath 162. A central axis of the sheath 162 and a central axis of the actuating member 150 are parallel with each other. The actuating member 150 is connected to the wire or rod connected to the movable handle 176. The actuating member 150 moves to the distal side and the proximal side in the sheath 162 along with the motion of the movable handle 176. Along with this movement, the second grasping member 130 changes its position in the rotary direction. A line connecting the first pivot 131 and the second pivot 132 is inclined relative to a longitudinal direction of the second grasping member 130. Therefore, the second grasping member 130 changes its position so as to open and close relative to the first grasping member 120.

In the present embodiment, an example in which the actuating member 150 passes into the sheath 162 is shown, but the configuration is not limited thereto. The actuating member 150 may have any configuration as long as it is disposed along the sheath 162 and moves along the longitudinal axis of the sheath 162 to thereby operate the second grasping member 130. For example, the actuating member 150 may be provided outside the sheath 162.

A third pivot 133 is provided at the distal portion of the second grasping member 130. A central portion of the swing member 140 is swingably connected by the third pivot 133 to a portion of the second grasping member 130 closer to the first grasping member 120. Namely, the second grasping member 130 and the swing member 140 are connected to each other so that an angle formed by the second grasping member 130 and the swing member 140 changes. In this embodiment, the swing member 140 is configured to swing in the same plane as the plane in which the second grasping member 130 rotates around the first pivot 131. In this manner, the swing member 140 is provided so as to face the first grasping member 120. At a time of using the treatment tool, the biological tissue as a treatment object is grasped between the first grasping member 120 and the second grasping member 130.

Because the swing member 140 swings, the treatment portion 110 is able to grasp the biological tissue with the same pressure on the distal side and the proximal side irrespective of the thickness of the biological tissue. Also, even if the thickness of the biological tissue differs between the distal side and the proximal side, the treatment portion 110 is able to grasp the biological tissue with the same pressure on the distal side and the proximal side. Applying uniform pressure to the biological tissue as a treatment object produces an effect in stable sealing and coagulation as well as in excision of the biological tissue.

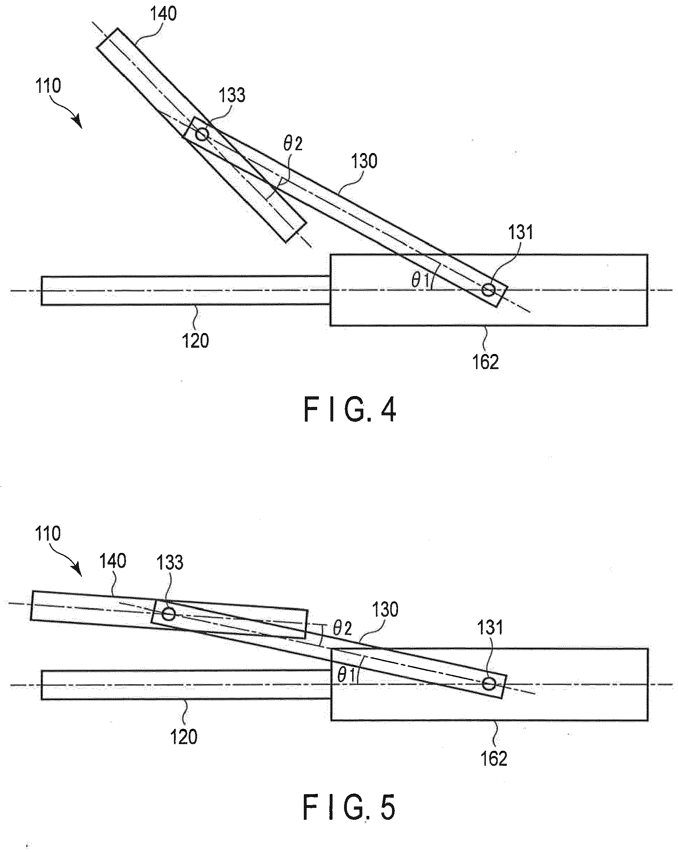

FIG. 4 shows a schematic diagram of the treatment portion 110. The first grasping member 120 protrudes from the distal portion of the sheath 162. The sheath 162 and the first grasping member 120 are arranged in a manner so that the central axis of the sheath 162 and the central axis of the first grasping member 120 are parallel with each other. The second grasping member 130 is attached to the sheath 162 and is rotatable around the first pivot 131. The swing member 140 is attached to the second grasping member 130 so as to swing around the third pivot 133.

When the plane of the paper of FIG. 4 is defined as a virtual plane, the longitudinal axes of the sheath 162 and the first grasping member 120 are on this virtual plane. Likewise, the longitudinal axes of the second grasping member 130 and the swing member 140 are also on this virtual plane. The second grasping member 130 rotates around the first pivot 131 on the virtual plane. The swing member 140 rotates around the third pivot 133 on the virtual plane.

An angle formed by the longitudinal axis of the first grasping member 120 and the longitudinal axis of the second grasping member 130 is set as a first angle θ1. The first angle θ1 takes a positive value in the opening direction, the state in which the first grasping member 120 and the second grasping member 130 are closed being set as an initial line (0°). An angle that the second grasping member 130 may form with the first grasping member 120, namely, a range of the first angle θ1, is determined suitably according to the design of the treatment portion 110. The range of the first angle θ1 is set as a first range, which includes a minimum value θ1min to a maximum value θ1max. The minimum value θ1min may be a negative value.

An angle formed by the longitudinal axis of the second grasping member 130 and the longitudinal axis of the swing member 140 is set as a second angle θ2. The second angle θ2 takes a positive value in a direction in which the proximal side of the swing member 140 moves toward the first grasping member 120, the state in which the second grasping member 130 and the swing member 140 are parallel with each other being set as an initial line (0°). An angle that the swing member 140 may form with the second grasping member 130, namely, a range of the second angle θ2, is also determined according to the design of the treatment portion 110. The range of the second angle θ2 is set as a second range, which includes a minimum value θ2min to a maximum value θ2max. The minimum value θ2min takes a negative value.

FIG. 5 shows a schematic diagram of an appearance of the treatment portion 110 in a closed state, for example, as in the case of grasping the biological tissue. When grasping the biological tissue, the first angle θ1 takes a small value according to the thickness of the biological tissue, as shown in FIG. 5. When grasping the biological tissue with the treatment portion 110, it is preferable to make the thickness of the biological tissue uniform and apply equal pressure to the biological tissue on the distal side and the proximal side of the treatment portion 110. Namely, it is preferable for the first grasping member 120 and the swing member 140 that pinch the biological tissue to form an angle close to parallel. In this case, the second angle θ2 takes a negative value, and an absolute value of the first angle θ1 and an absolute value of the second angle θ2 take a close value.

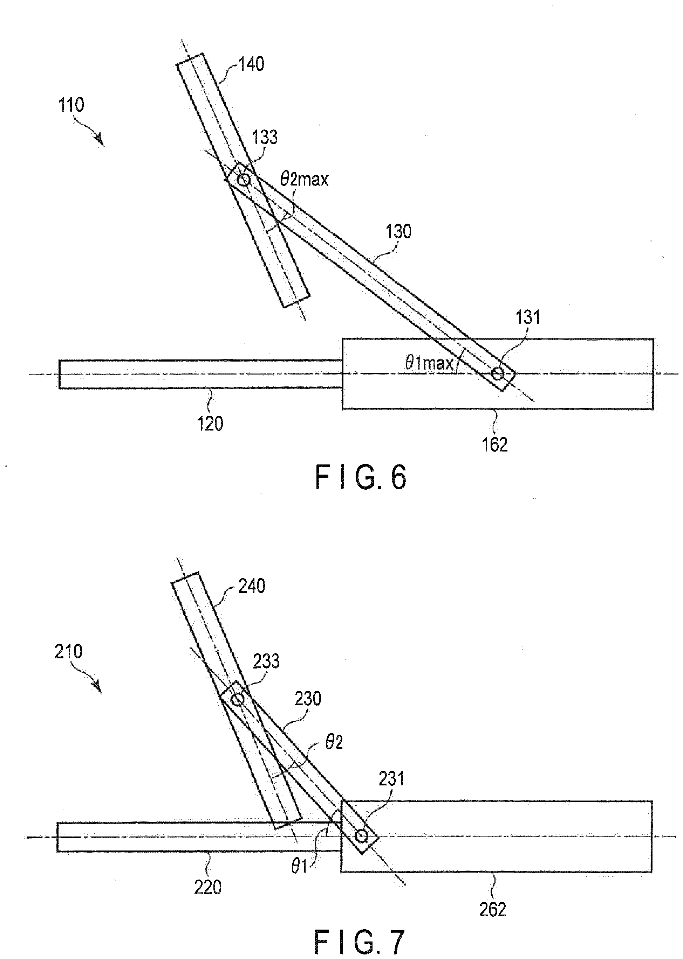

FIG. 6 shows a schematic diagram of an appearance of the treatment portion 110 in an opened state before grasping the biological tissue. Before grasping the biological tissue, the second grasping member 130 opens widely so that the biological tissue is easily positioned between the first grasping member 120 and the swing member 140, as shown in FIG. 6. Namely, the first angle θ1 takes a large value. The first angle θ1 becomes, for example, the maximum value θ1max. In addition, the swing member 140 inclines significantly at this time so that the biological tissue is easily positioned between the first grasping member 120 and the swing member 140. Namely, the second angle θ2 takes a large value. The second angle θ2 becomes, for example, the maximum value θ2max.

In the present embodiment, in the case where the second angle θ2 is the maximum value θ2max, the proximal side of the swing member 140 does not contact the first grasping member 120 at least when the first angle θ1 is the maximum value θ1max. Preferably, even when the first angle θ1 is smaller than the maximum value θ1max, the proximal side of the swing member 140 does not contact the first grasping member 120 despite the second angle θ2 being the maximum value θ2max. Also, if the maximum value θ2max of the second angle θ2 is not very large, the proximal side of the swing member 140 may not contact the first grasping member 120 even when the first angle θ1 is zero, namely, when the first grasping member 120 and the second grasping member 130 are parallel to each other and closed. In this manner, the treatment portion 110 may be configured so that when the second angle θ2 takes the maximum value θ2max, the proximal side of the swing member 140 does not contact the first grasping member 120 even if the first angle θ1 is zero.

FIG. 7 shows a schematic diagram according to a comparative example. A sheath 262, a first grasping member 220, and a swing member 240 of the comparative example shown in FIG. 7 are not respectively different from the sheath 162, the first grasping member 120, and the swing member 140 of the above-described embodiment. On the other hand, a length of a second grasping member 230 of the comparative example shown in FIG. 7 is smaller than that of the second grasping member 130 of the above-described embodiment. In a manner similar to the above-described embodiment, the second grasping member 230 is rotatably supported by a first pivot 231 that is provided to the sheath 262 on the proximal side of the second grasping member 230. The swing member 240 is rotatably supported by a third pivot 233 that is provided at the distal side of the second grasping member 230 near the center of the swing member 240. In the comparative example, since the second grasping member 230 is short, the first pivot 231 is positioned at the distal side as compared with the case of the above-described embodiment, and the maximum value θ1max of the first angle θ1 is increased, thereby increasing an opening angle between the first grasping member 220 and the swing member 240. However, the proximal end of the swing member 240 contacts the first grasping member 220, as shown in FIG. 7.

If the proximal portion of the swing member 240 contacts the first grasping member 220, the swing member 240 and the first grasping member 220 may break. Especially, when the proximal side of the first grasping member 220 that serves to support a greater load is damaged, the first grasping member 220 may break. The proximal end of the first grasping member 220 is more likely to be damaged when the first grasping member 220 vibrates at an ultrasonic frequency.

Accordingly, the present embodiment is designed so that the swing member 140 and the first grasping member 120 do not come into contact with each other at least when the first angle θ1 is at its maximum in the state where the second angle θ2 is the maximum value θ2max, where the proximal side of the swing member 140 is closest to the first grasping member 120. This prevents breaking of the first grasping member 120 and the swing member 140 caused by the proximal side of the swing member 140 contacting the first grasping member 120.

Especially in the case of the structure in which the second grasping member 130 attached to the sheath 162 is opened and closed by the motion of the actuating member 150 that is disposed along the sheath 162 and moves in parallel with the central axis of the sheath 162, as described in the present embodiment, the swing member 140 is likely to contact the first grasping member 120 depending on the design. The present embodiment is designed so that the swing member 140 does not contact the first grasping member 120, even in the structure having such actuating member 150.

Hereinafter, some modifications of the above-described embodiment are described. Differences from the above-described embodiment are described below, and descriptions of the same parts, for which the same symbols are used, are omitted.

### [First Modification]

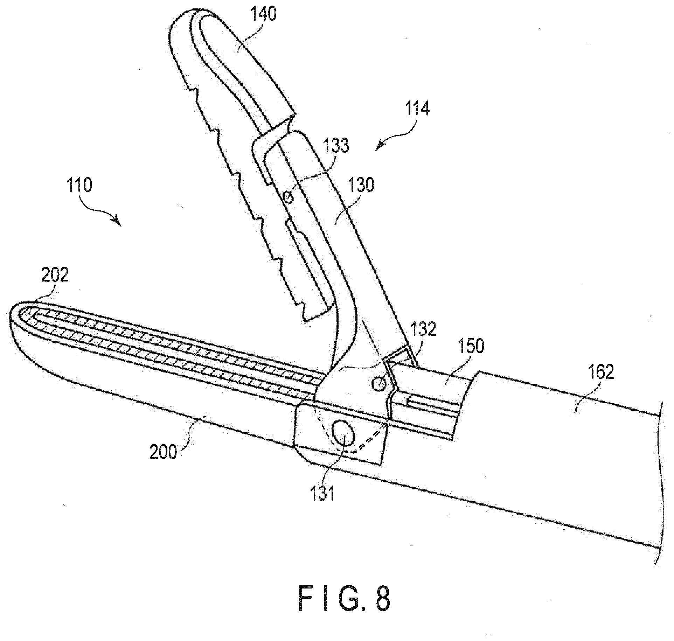

The above-described embodiment shows the example in which the treatment tool 100 treats the biological tissue by ultrasonic vibration and high-frequency power. However, any energy source may be employed as the energy source for treating the biological tissue. For example, the treatment tool may use only ultrasonic vibration to treat the biological tissue, or use only high-frequency power to treat the biological tissue, as shown in FIG. 8.

In FIG. 8, the jaw 114 and a jaw member 200 are provided as the treatment portion 110 corresponding to an end effector. The swing member 140 functions as an electrode that outputs a high-frequency current; the jaw member 200 is provided in a manner facing the swing member 140; and an electrode 202 is provided in a manner facing the swing member 140. The electrode 202 functions as an electrode having a potential different from that of the swing member 140. In the case of the example described in FIG. 8, even if the swing member 140 inclines to the second grasping member 130, by adopting the structure of the above-described embodiment, the swing member 140 does not short-circuit against the electrode 202. Therefore, the electrode 202 or the swing member 140 does not break due to short circuit.

In addition, the treatment tool may use heat generated by a heater provided to the probe to treat the biological tissue, or use other energy to treat the biological tissue. A combination of two or more of ultrasonic vibration, high-frequency power, heat generated by a heater, and other energy may also be used to treat the biological tissue. The treatment tool 100 may be one that physically treats the biological tissue, such as one that includes a stapler or one that includes a cutter with a blade, or may be a combination of these.

### [Second Modification]

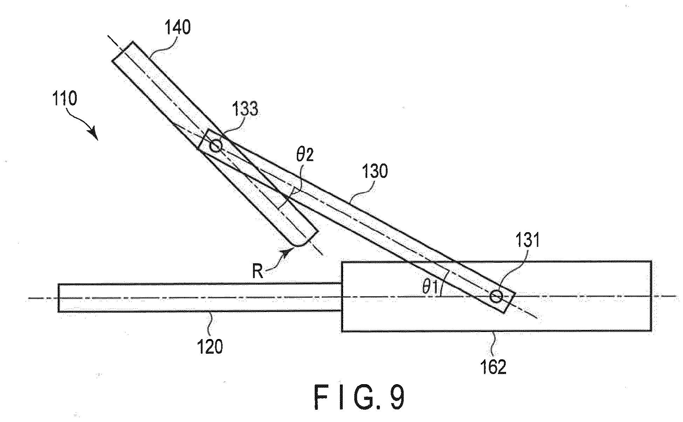

FIG. 9 shows a schematic diagram of the configuration of the treatment portion 110 according to the second modification. In this modification, the portion R on the proximal side of the swing member 140 that faces the first grasping member 120 is chamfered, as shown in FIG. 9, so that the proximal side of the swing member 140 is less likely to contact the first grasping member 120. Chamfering the portion in this manner makes the first grasping member 120 and the swing member 140 less likely to contact each other.

### [Third Modification]

A case where the first grasping member 120 and the swing member 140 are opened and the biological tissue is inserted therebetween is now considered. When the first grasping member 220 and the swing member 240 are configured to contact each other, as in the case of the comparative example shown in FIG. 7, it is hard to push the biological tissue as a treatment object further toward the proximal side than the swing member 240 because the biological tissue touches the swing member 240. By contrast, in the case of the above-described embodiment, there is a gap between the first grasping member 120 and the swing member 140. Therefore, the biological tissue is sometimes pushed further through this gap toward the proximal side than the swing member 140. When the biological tissue is positioned further toward the proximal side than the end of the swing member 140, treatment is not performed in this position.

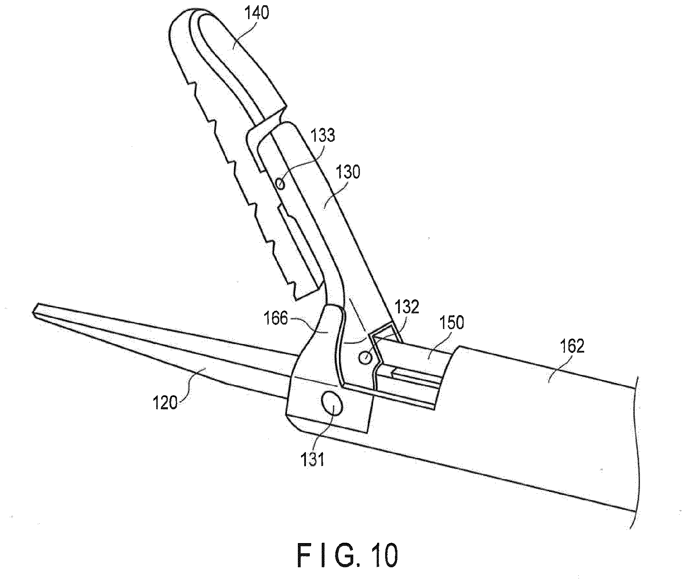

Accordingly, in this modification, a stopper 166 is provided at the distal side of the sheath 162, so that the biological tissue is not positioned further toward the proximal side than the swing member 140, as shown in FIG. 10.

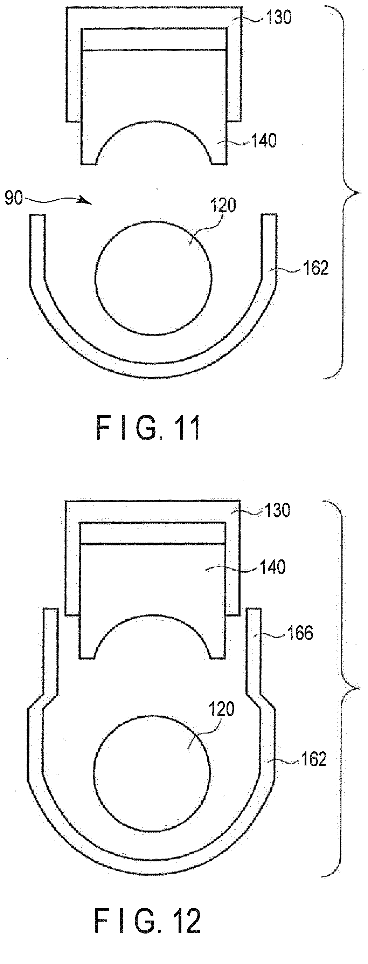

The stopper 166 is further described below with reference to FIGS. 11 and 12. FIG. 11 is a schematic diagram of the configuration of the treatment portion 110 according to the above-described embodiment seen from the distal side. There is a gap 90 between the first grasping member 120 and the swing member 140. The biological tissue is sometimes positioned further toward the proximal side than the swing member 140 by passing through the gap 90. Accordingly, in this modification, the stopper 166, which extends the sheath 162 toward the swing member 140, is provided so as to block the gap, as shown in the schematic diagram of FIG. 12. The biological tissue is not inserted further toward the proximal side than the stopper 166. As a result, the biological tissue is certainly grasped by the first grasping member 120 and the swing member 140 and treated.

In this description, an example in which the stopper 166 is integrally formed with the sheath 162 is shown. However, the stopper is not limited thereto. The stopper 166 may be formed separately from the sheath 162 and attached to the sheath 162.

### [Fourth Modification]

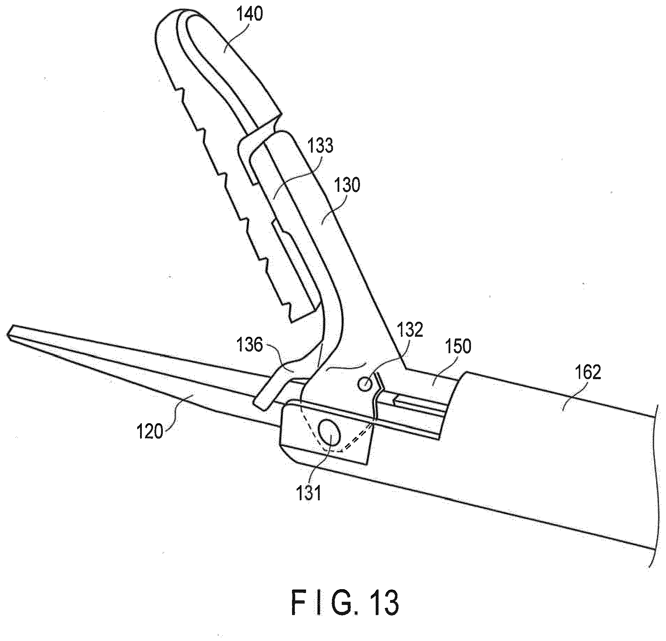

In this modification as well, a stopper is provided so as to prevent the biological tissue from being positioned further toward the proximal side than the end of the swing member 140, in a manner similar to the third modification. FIG. 13 schematically illustrates the treatment portion 110 according to this modification. In this modification, a stopper 136 is provided to the second grasping member 130 so as to block the gap between the first grasping member 120 and the swing member 140, as shown in FIG. 13.

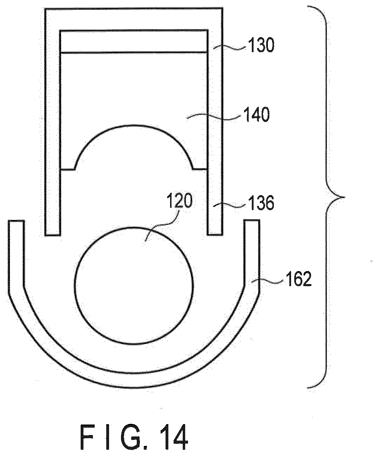

The stopper 136 is further described below with reference to FIG. 14. FIG. 14 is a schematic diagram of the configuration of the treatment portion 110 according to this modification. In this modification, the stopper 136, which extends the second grasping member 130 toward the first grasping member 120, is provided so as to block the gap, as shown in FIG. 14. The biological tissue is not inserted further toward the proximal side than the stopper 136. As a result, the biological tissue is certainly grasped by the first grasping member 120 and the swing member 140 and treated.

In this description, an example in which the stopper 136 is integrally formed with the second grasping member 130 is shown. However, the stopper is not limited thereto. The stopper 136 may be formed separately from the second grasping member 130 and attached to the second grasping member 130.

The above-described embodiment and the configuration of each modification may be employed in suitable combination.

## Claims

1. A treatment tool comprising:
a sheath that is provided in a manner so that a longitudinal axis thereof is arranged on a virtual plane;
a first grasping member that protrudes from a distal portion of the sheath along the longitudinal axis;
a second grasping member that is rotatably provided to the sheath on the virtual plane so that a first angle, which is an angle formed with the first grasping member, changes within a predetermined first range;
a swing member that is provided to the second grasping member so as to swing on the virtual plane and so that a second angle, which is an angle formed with the second grasping member, changes within a predetermined second range; and
an actuating member that is disposed along the sheath, is connected to the second grasping member, and moves along the longitudinal axis to thereby displace the second grasping member so as to change the first angle,
wherein when the second angle is at its maximum within the second range, there is a first angle at which the first grasping member and the swing member do not contact each other within the first range.

2. The treatment tool according to claim 1, wherein the first grasping member and the swing member do not contact each other when the second angle is at its maximum within the second range even when the first angle is not at its maximum within the first range.

3. The treatment tool according to claim 1, wherein the first grasping member and the swing member do not contact each other when the second angle is at its maximum within the second range even when the first angle is zero.

4. The treatment tool according to claim 1, further comprising a stopper that is provided so as to prevent biological tissue as a treatment object inserted between the first grasping member and the swing member from being positioned further toward a proximal side than an area where the first grasping member and the swing member face each other.

5. The treatment tool according to claim 4, wherein the stopper is provided to the sheath.

6. The treatment tool according to claim 4, wherein the stopper is provided to the second grasping member.

7. The treatment tool according to claim 1,
wherein an ultrasonic transducer is directly or indirectly connected to the first grasping member, and
wherein the treatment tool is configured to treat biological tissue sandwiched between the first grasping member and the swing member by ultrasonic vibration of the first grasping member.

8. The treatment tool according to claim 1,
wherein the first grasping member and the swing member are electrically connected to a power supply that outputs high-frequency power, and
wherein the treatment tool is configured to treat biological tissue sandwiched between the first grasping member and the swing member by supplying high-frequency power from the first grasping member and the swing member to the biological tissue.

9. The treatment tool according to claim 1,
wherein an ultrasonic transducer is directly or indirectly connected to the first grasping member,
wherein the first grasping member and the swing member are configured to be electrically connected to a power supply that outputs high-frequency power,
wherein the treatment tool is configured to treat biological tissue sandwiched between the first grasping member and the swing member by ultrasonic vibration of the first grasping member, and
wherein the treatment tool is configured to treat biological tissue sandwiched between the first grasping member and the swing member by supplying high-frequency power from the first grasping member and the swing member to the biological tissue.
